# EUROPÄISCHE PATENTANMELDUNG

(11) **EP 1 059 303 A2**
(43) Veröffentlichungstag der Anmeldung: **13.12.2000**
(21) Anmeldenummer: 99124427.8
(22) Anmeldetag: 08.12.1999
(51) Int. Cl.: C07K 14/76

(54) **Verfahren zum Auflösen von Albuminflocken in einer Flüssigkeit sowie Einrichtung zur Durchführung des Verfahrens**

(30) Priorität: 17.12.1998 DE 19858188
(71) Anmelder: Aventis Behring Gesellschaft mit beschränkter Haftung, 35002 Marburg (DE)
(72) Erfinder: Nettelhoff, Hubert, Dr., 35041 Marburg (DE); Römisch, Jürgen, Dr., 35041 Marburg (DE)

(57) **Zusammenfassung**

Die Erfindung schlägt ein Verfahren sowie eine Einrichtung zum Auflösen von Albuminflocken in einer Flüssigkeit, die in einem Behältnis abgefüllt ist, vor.

Gemäß dem erfindungsgemäßen Verfahren ist vorgesehen, daß die Flocken relativ zur Flüssigkeit bewegt werden. Die Einrichtung zur Durchführung des Verfahrens weist eine konstruktive Ausgestaltung auf, die es gestattet, mehrere Behältnisse (6) gleichzeitig nach dem erfindungsgemäßen Verfahren zu behandeln, wobei die Einrichtung einen oder mehrere Motoren (10) zum Antrieb der Konstruktionselemente (3, 4, 7, 8, 9) aufweist, welche die Behältnisse (6) während der Behandlung in eine rotierende oder translatorische Bewegung versetzen.

## Beschreibung

Die Erfindung betrifft ein Verfahren zum Auflösen von Albuminflocken in einer Flüssigkeit sowie eine Einrichtung zur Durchführung des Verfahrens.

In den pasteurisierten Endabfüllungen von Humanalbumin wird häufig Flockenbildung beobachtet. Diese flockenhaltigen Präparationen sind nicht verkäuflich und werden daher in der visuellen Endkontrolle der Fertigung aussortiert. Die beanstandeten Behältnisse, insbesondere Flaschen, werden manuell geöffnet und der Inhalt wird in einen Sammelbehälter entleert. Anschließend wird die Albuminlösung zwecks Flockenabtrennung filtriert. Das Filtrat wird wie im vorhergehenden Fertigungsprozeß wieder in Flaschen abgefüllt und verschlossen, pasteurisiert und optisch kontrolliert. Der bei weitem überwiegende Teil der aufgearbeiteten Albuminlösung ist frei von Flocken und kann in der Verpackungslinie weiterverarbeitet werden. - Die Aufarbeitung von Flockenware ist personalintensiv, verursacht zusätzliche Kosten durch den Verlust von Wertstoff und den Verbrauch von Primärpackmitteln und Hilfsstoffen und erhöht die Menge anfallenden Mülls in Form benutzter Primärpackmittel, Filter und Waschlösungen. In der visuellen Kontrolle falschnegativ bewertete Flockenware wird vom Kunden beanstandet, führt zu Retouren und kann darüber hinaus das Firmenimage am Markt negativ beeinflussen.

Es ist Aufgabe der vorliegenden Erfindung, ein einfaches, gut wirksames Verfahren zum Auflösen von Albuminflocken in einer Flüssigkeit sowie eine baulich einfach gestaltete Einrichtung zur Durchführung des Verfahrens anzugeben.

Die Erfindung schlägt ein Verfahren zum Auflösen von Albuminflocken in einer Flüssigkeit, die in einem Behältnis abgefüllt ist, vor, derart, daß die Flocken und die Flüssigkeit relativ zueinander bewegt werden.

Im Sinne der Erfindung ist der entscheidende Faktor für die Kinetik der Auflösung der Flocken in der Flüssigkeit die Relativbewegung der Flocken zum Flüssigkeitsbulk. Bei Flüssigkeiten, die in Flaschen abgefüllt sind, ist eine hohe Relativgeschwindigkeit insbesondere durch Rotation der Flaschen um ihre Längsachse mit hoher Drehzahl gewährleistet. Die Geschwindigkeitsdifferenz ist aufgrund der Massenträgheit am größten kurz nach Beginn der Rotation und direkt nach dem Abbremsen der Flaschen. Die größten Erfolgsaussichten für das Auflösen von Albuminflocken bestehen damit im schnellen Beschleunigen der Flaschen auf hohe Drehzahlen und anschließendem zügigen Abbremsen.

Vorzugsweise werden die Behältnisse liegend rotiert, da hierdurch eine Benetzung der gesamten inneren Oberfläche, einschließlich des Verschlußstopfens, Verschlußdeckels oder dergleichen gewährleistet ist.

Das mehrmalige Beschleunigen der Flaschen kann bei derselben Drehrichtung oder auch bei entgegengesetzter Drehrichtung erfolgen, bevorzugt bei entgegengesetzter Drehrichtung.

Die Erfindung ist nicht darauf beschränkt, daß die Behältnisse bzw. Flaschen rotieren, sondern es kann das Auflösen der Albuminflocken durch ausschließlich translatorische Bewegung mit Beschleunigungs- und Bremsabschnitten, gegebenenfalls durch eine translatorische Bewegung in entgegengesetzten Richtungen erfolgen.

Die Einrichtung zur Durchführung des erfindungsgemäßen Verfahrens weist zumindest folgende Merkmale auf:
- eine konstruktive Ausgestaltung, die es gestattet, mehrere Behältnisse gleichzeitig nach dem erfindungsgemäßen Verfahren zu behandeln,
- einen oder mehrere Motoren zum Antrieb der Konstruktionselemente, welche die Behältnisse während der Behandlung in eine rotierende oder translatorische Bewegung versetzen.

Es ist insbesondere daran gedacht, daß die Behältnisse rotationssymmetrisch, insbesondere als Flaschen ausgebildet sind.

Gemäß einer besonderen Ausgestaltung der erfindungsgemäßen Einrichtung ist vorgesehen, daß mehrere Behandlungsstellen zu einer mehrstelligen Einheit gekoppelt sind. Die Anordnung der Behandlungsstellen erfolgt bevorzugt nebeneinander in einer Ebene. Die Auflageelemente der Behandlungsstellen sind in ihrer Länge und in ihrem Abstand so zu wählen, daß die Behältnisse zwischen jeweils benachbarten Aufnahmeelementen sicher und bevorzugt in liegender Position aufgenommen werden können. Die Auflagen für die zu behandelnden Behältnisse sind rotationssymmetrisch bevorzugt als Rollen, Zylinder oder Hülsen ausgeführt. Die Enden der Achsen der Auflageelemente werden in geeigneten Lagern, z. B. Wälz- oder Gleitlagern aufgenommen. Die Behandlungsstellen sind aus angetriebenen und losen Rollen aufgebaut. Zur Durchführung des erfindungsgemäßen Verfahrens liegen die Behältnisse auf jeweils einer angetriebenen und einer lose gelagerten Rolle auf. Gemäß einer bevorzugten Gestaltung ist vorgesehen, die Teilung der Behandlungseinheit und die Anordnung der angetriebenen und der losen Rollen derart zu wählen, daß jeweils zwei Behältnisse von jeweils einer angetriebenen Rolle in Rotation versetzt werden können. Die Beschaffenheit der angetriebenen Rollen wird derart gewählt, daß ein ausreichender Reibwert zum Antrieb der Behältnisse durch die Rollen resultiert.

Dies erfolgt durch die Ausführung der angetriebenen Rollen aus einem geeigneten Material oder durch eine Beschichtung der Rollen mit einem geeigneten Material oder durch das Aufbringen von geeigneten Elementen, beispielsweise O-Ringen oder Rundschnürringen aus einem geeignetem Material, vorzugsweise aus einem Elastomer. Die Kopplung von mehreren angetriebenen Rollen untereinander und mit dem Antriebsmotor erfolgt über eine geeignete kraftschlüssige Verbindung, beispielsweise über Verbindungselemente wie Zahnräder, Ketten, Antriebsriemen, vorzugsweise über Zahnriemen. Als Antriebsmotor dient ein Elektromotor, vorzugsweise mit umschaltbarem Drehsinn und mit regelbarer Drehzahl. Einschaltdauer, Drehsinn und Drehzahl des Motors werden vorteilhaft mittels einer automatisch arbeitenden Steuereinheit gesteuert.

Die erfindungsgemäße Einrichtung ist nicht auf eine Ausführung mit sechs Behandlungsstellen beschränkt.

Es ist auch an die Anordnung mehrerer Teilbehandlungseinheiten übereinander, nebeneinander oder hintereinander gedacht.

Es ist auch an die Anordnung mehrerer einstelliger oder zweistelliger Behandlungseinheiten mit jeweils separatem Antriebsmotor gedacht.

Es ist auch an die Kombination der Behandlungseinheiten mit automatisch arbeitenden Aufgabe- und Entnahmestationen gedacht.

Es ist auch an die Kombination einer Behandlungseinheit mit einer Einrichtung zum gleichzeitigen Weitertransport der Behältnisse gedacht.

Weitere Merkmale der Erfindung sind in den Unteransprüchen, der Beschreibung der Figuren und der Figur selbst dargestellt, wobei bemerkt wird, daß alle Einzelmerkmale und alle Kombinationen von Einzelmerkmalen erfindungswesentlich sind.

In der einzigen Figur ist anhand einer schematischen Darstellung eine Ausführungsform der Erfindung dargestellt, ohne hierauf beschränkt zu sein.

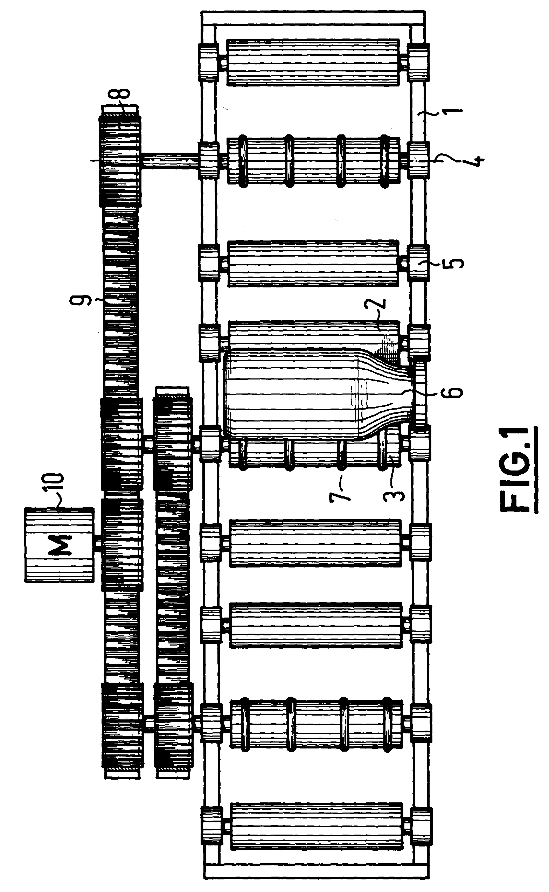

Die Figur 1 zeigt eine Einrichtung zum Auflösen von Albuminflocken in einer Flüssigkeit, die in Flaschen abgefüllt ist. Die Einrichtung ist in einer Draufsicht dargestellt.

Ein Rahmen 1 nimmt insgesamt neun lose Rollen 2 und angetriebene Rollen 3 auf, die parallel zueinander und in einer Ebene angeordnet sind. Die Achsen 4 der Rollen 2 und 3 werden beiderseits der Rollenenden in Wälzlagern 5 geführt. Die Wälzlager 5 sind in entsprechenden Aussparungen des Rahmens 1 fixiert. Die Länge der Rollen 2 und 3 ist so gewählt, daß die zu behandelnden Flaschen 6 liegend von der Einrichtung parallel zu den Rollen 2 und 3 in Längsrichtung aufgenommen werden können.

Die angetriebenen Rollen 3 der Einrichtung sind mit jeweils vier O-Ringen 7 aus einem Elastomermaterial versehen, welche durch entsprechende Aussparungen der angetriebenen Rollen 3 fixiert sind. Durch den erhöhten Reibwert der O-Ringe 7 wird zuverlässig eine Übertragung der Rotationsbewegung der angetriebenen Rollen 3 auf die zu behandelnden Flaschen 6 erreicht. Die Anordnung der angetriebenen Rollen 3 und der losen Rollen 2 in der Einrichtung ist so gewählt, daß jeweils zwei Flaschen 6 von jeweils einer angetriebenen Rolle 3 in Rotation versetzt werden können. Hierbei liegt jede Flasche 6 auf jeweils einer angetriebenen Rolle 3 und einer losen Rolle 2 auf. Die Abstände der Rollen 2 und 3 sind so gewählt, daß die Flaschen 6 nicht gegeneinanderstoßen.
Die auf der Rückseite der Einrichtung verlängerten und durch die Lagerung 5 hindurchgeführten Achsen 4 der angetriebenen Rollen 3 sind mit Zahnscheiben 8 versehen und kraftschlüssig mit diesen verbunden. Die Übertragung der Drehbewegung zwischen den Zahnscheiben 8 jeweils benachbarter angetriebener Rollen 3 erfolgt mittels Zahnriemen 9.

Die Achse 4 einer der äußeren angetriebenen Rollen 3 ist auf der Rückseite der Einrichtung mit einer weiteren Zahnscheibe 8 versehen und mit dieser drehfest verbunden. Diese Zahnscheibe 8 ist über einen Zahnriemen 9 mit einer Zahnscheibe 8 verbunden, welche kraftschlüssig mit der Abtriebswelle eines Elektromotors 10 verbunden ist. Der Antriebsmotor 10 ist mit umschaltbarem Drehsinn und regelbarer Drehzahl ausgeführt. Die Ansteuerung des Motors 10 hinsichtlich dessen Einschaltdauer, Drehsinns und Drehzahl erfolgt mittels einer entsprechenden Steuereinheit. Hierdurch wird eine Automatisierung des Beschleunigens und Abbremsens der Flaschen, sowie des Drehrichtungswechsels zur Optimierung der Flockenauflösung ermöglicht.

Die Drehzahl der Flaschen liegt vorzugsweise bei 200 pro Minute bis etwa 560 pro Minute, wobei die Drehzahl nach oben durch den Beginn der Schaumbildung begrenzt ist.

## Patentansprüche

1. Verfahren zum Auflösen von Albuminflocken in einer Flüssigkeit, die in einem Behältnis abgefüllt ist, derart, daß die Flocken relativ zur Flüssigkeit bewegt werden.

2. Verfahren nach Anspruch 1, wobei die Flocken relativ zur Flüssigkeit durch wechselnde Beschleunigung des Behälters bewegt werden.

3. Verfahren nach Anspruch 2, wobei das Behältnis rotierend bewegt wird.

4. Verfahren nach Anspruch 3, wobei das Behältnis auf hohe Drehzahlen, vorzugsweise 200 bis 560 pro Minute, beschleunigt und zügig abgebremst wird.

5. Verfahren nach einem der Ansprüche 2 bis 4, wobei das Behältnis in entgegengesetzten Richtungen bewegt wird.

6. Verfahren nach einem der Ansprüche 2 bis 5, wobei das Behältnis liegend bewegt wird.

7. Einrichtung zur Durchführung des Verfahrens nach einem der Ansprüche 1 bis 6, mit folgenden Merkmalen,
- einer konstruktiven Ausgestaltung, die es gestattet, mehrere Behältnisse (6) gleichzeitig nach dem erfindungsgemäßen Verfahren zu behandeln,
- einem oder mehreren Motoren (10), zum Antrieb der Konstruktionselemente (3), welche die Behältnisse (6) während der Behandlung in eine rotierende oder translatorische Bewegung versetzen.

8. Einrichtung nach Anspruch 7, wobei die Behältnisse (6) rotationssymmetrisch, insbesondere als Flaschen (6) ausgebildet sind.

9. Einrichtung nach Anspruch 7 oder 8, wobei mehrere Behandlungsstellen bevorzugt nebeneinander in einer Ebene angeordnet sind.

10. Einrichtung nach einem der Ansprüche 7 bis 9, wobei die Behältnisse (6) zwischen jeweils benachbarten Aufnahmeelementen (2, 3) bevorzugt in liegender Position aufgenommen werden.

11. Einrichtung nach Anspruch 7, wobei die Auflagen (2, 3) für die zu behandelnden Behältnisse (6) rotationssymmetrisch, bevorzugt als Rollen, Zylinder oder Hülsen (2, 3) ausgeführt sind und die Achsen (4) der Auflagen (2, 3) in geeigneten Lagern (5) aufgenommen werden.

12. Einrichtung nach Anspruch 10 oder 11, wobei die Behältnisse (6) auf jeweils einer angetriebenen Rolle (3) und einer lose gelagerten Rolle (2) aufliegen.

13. Einrichtung nach Anspruch 12, wobei jeweils zwei Behältnisse (6) von jeweils einer angetriebenen Rolle (3) in Rotation versetzt werden können.

14. Einrichtung nach einem der Ansprüche 11 bis 13, wobei durch die Ausführung der angetriebenen Rollen (3) aus einem geeigneten Material oder durch eine Beschichtung der Rollen (3) mit einem geeigneten Material oder durch das Aufbringen von geeigneten Elementen (7) aus einem geeignetem Material, vorzugsweise aus einem Elastomer, ein ausreichender Reibwert zum Antrieb der Behältnisse (6) durch die Rollen (3) resultiert.

15. Einrichtung nach einem der Ansprüche 7 bis 14, wobei die Kopplung von mehreren angetriebenen Rollen (3) untereinander und mit dem Antriebsmotor (10) über eine geeignete kraftschlüssige Verbindung, beispielsweise über Verbindungselemente wie Zahnräder, Ketten, Antriebsriemen, vorzugsweise über Zahnriemen (9) erfolgt.

16. Einrichtung nach Anspruch 7, wobei als Antriebsmotor (10) ein Elektromotor (10) dient, vorzugsweise mit umschaltbarem Drehsinn und mit regelbarer Drehzahl, dessen Einschaltdauer, Drehsinn und Drehzahl vorteilhaft mittels einer automatisch arbeitenden Steuereinheit gesteuert werden.
